Europäisches Patentamt

European Patent Office

Office européen des brevets

(11) Veröffentlichungsnummer: **0 272 638 B1**

(12) **EUROPÄISCHE PATENTSCHRIFT**

(45) Veröffentlichungstag der Patentschrift: **20.05.92**

(21) Anmeldenummer: **87118771.2**

(22) Anmeldetag: **17.12.87**

Die Akte enthält technische Angaben, die nach dem Eingang der Anmeldung eingereicht wurden und die nicht in dieser Patentschrift enthalten sind.

(51) Int. Cl.5: **A61K 31/415**, //(A61K31/415, 31:165)

(54) **Analgetisches Präparat.**

(30) Priorität: **24.12.86 CH 5183/86**

(43) Veröffentlichungstag der Anmeldung:
**29.06.88 Patentblatt 88/26**

(45) Bekanntmachung des Hinweises auf die Patenterteilung:
**20.05.92 Patentblatt 92/21**

(84) Benannte Vertragsstaaten:
**BE CH DE FR GB IT LI NL**

(56) Entgegenhaltungen:

**CHEMICAL ABSTRACTS, Band 69, 14. Oktober 1968, Seite 6163, Zusammenfassung Nr. 66058f, Columbus, Ohio, US; H. NIWA et al.: "Combination of drugs. II. Effect of p-hydroxyacetanilide on the metabolism of pyrazolones", & YAKUGAKU ZASSHI 1968, 88(5), 542-8**

**Idem**

**DIALOG, Nr. 0104608, International Pharmaceutical Zusammenfassung Nr. 21-03214;**

**B.W. MULLER et al.: "Polymorphism and alteration of shape of propyphenazone", & ACTA PHARM. TECHNOL. 28:97-102, (2), 1982**

**Idem**

**UNLISTED DRUGS, Band 38, Nr. 1, Januar 1986, Seite 13j, Chatman, New Jersey, US**

**Lancet 1989 381**

(73) Patentinhaber: **F. HOFFMANN-LA ROCHE AG**
**Postfach 3255**
**CH-4002 Basel(CH)**

(72) Erfinder: **Kunovits, Georg**
**Rüttiweg 32**
**CH-4144 Arlesheim(CH)**

(74) Vertreter: **Lederer, Franz, Dr. et al**
**Patentanwalt Dr. Franz Lederer Lucile-Grahn-Strasse 22**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.08/2.19/2.0)

**Beschreibung**

Die Erfindung betrifft ein analgetisches Präparat das, ausser üblichen Hilfsstoffen, ausschliesslich aus Paracetamol und Propyphenazon besteht. Es enthält somit neben den beiden genannten Wirkstoffen keine weiteren Wirkstoffe, insbesondere kein Coffein. Vorzugsweise beträgt das Gewichtsverhältnis Paracetamol zu Propyphenazon 5 zu 3.

In Acta Pharm. Technol. 28, 97-102 (1982) werden Zustandsdiagramme binärer Propyphenazon-Paracetamol-Gemische beschrieben. Das Dokument Unlisted Drugs, Band 38, Nr. 1, Januar 1986, Seite 13 j, Chatman, N.Y., USA, erwähnt das Analgetikum "Saridon Neu", das Propyphenazon, Paracetamol und Coffein enthält."

Die analgetischen Präparate der vorliegenden Erfindung werden vorzugsweise oral, z.B. als Tabletten, Kapseln oder Dragées, verabreicht. Man kann sie aber auch rektal als Suppositorien verabreichen. Beim Erwachsenen erfolgt die Behandlung eines Schmerzanfalls zweckmässig durch Verabreichung von einer bis sechs, vorzugsweise von zwei Dosierungseinheiten, die 300 bis 500, vorzugsweise 400 mg der Wirkstoffe Paracetamol und Propyphenazon pro Dosierungseinheit enthalten.

Die analgetischen Präparate der vorliegenden Erfindung entfalten einen ausgeprägten analgetischen Gesamteffekt, der rasch einsetzt und mehrere Stunden lang anhält. Als Indikationen dieser Präparate können Schmerzzustände, wie Kopfschmerzen, Migräne, Zahnschmerzen, Menstruationsbeschwerden, Neuralgien und Neuritiden, postoperative Schmerzen, sowie Fiebersenkung bei Infektionskrankheiten genannt werden.

Hundert Patienten mit postoperativen Schmerzen wurden zwei Tabletten enthaltend 250 mg Paracetamol und 150 mg Propyphenazon verabreicht. 30 Minuten nach der Verabreichung waren bei 28% der Patienten die Schmerzen verschwunden und bei 66% der Patienten war eine wesentliche Besserung festzustellen, während nur bei 6% der Patienten kein Besserung eingetreten war.

Als übliche Hilfsstoffe in den erfindungsgemässen oral verabreichbaren Präparaten sind Füllmittel, wie Cellulose, vorzugsweise mikrokristalline Cellulose, Milchzucker oder Mannitol; Bindemittel, wie Polyvinylpyrrolidon, Methylcellulose oder vorzugsweise Hydroxypropylmethylcellulose; Sprengmittel, wie Natriumcarboxymethylstärke oder vorzugsweise Polyvinylpolypyrrolidon; Spreng- und Füllmittel, wie Kartoffel- oder Maisstärke oder physikalisch modifizierte Stärke; Gleitmittel, wie Talk oder Magnesiumstearat, und Fliessregulierungsmittel, wie Kieselsäure, zu nennen. Als übliche Hilfsstoffe für Dragées kann man Zucker, Titandioxyd und Talk für den Dragéekern, und Maisstärke, Gummi arabicum, Paraffin, Paraffinöl und ein Farbstoff für den Dragéemantel nennen. Als übliche Hilfsstoffe für Suppositorien kommen pflanzliche oder synthetische Fettkörper, wie Kakaobutter oder Massa estarinum, und halbsynthetische Glyceride in Frage.

Die Herstellung der erfindungsgemässen Präparate kann nach an sich bekannten Methoden durch Vermischen der Aktiv-und der Hilfsstoffe erfolgen.

Beispiel 1

Tabletten folgender Zusammensetzung wurden nach dem Doppelgranulat-Verfahren hergestellt. Dabei wurden die zwei Wirkstoffe zunächst mit einem Teil der Hilfsstoffe und dann unter Hinzufügen eines Trockenzuschlages der restlichen Hilfsstoffe granuliert.

| | |
|---|---|
| Paracetamol | 250,0 mg |
| Propyphenazon | 150,0 mg |
| AVICEL PH-102® (mikrokristalline Cellulose) | 120,0 mg |
| Hydroxypropylmethylcellulose 2910/3 cP | 8,0 mg |
| Hydroxypropylmethylcellulose 2910/15 cP | 4,0 mg |
| POLYPLASDONE XL® (Polyvinylpolypyrrolidon) | 10,0 mg |
| Maisstärke weiss | 42,0 mg |
| Talk | 9,8 mg |
| Magnesiumstearat | 5,0 mg |
| Kieselsäure AEROSIL® 200 | 1,2 mg |
| Tablettengewicht | 600,0 mg |

EP 0 272 638 B1

Beispiel 2

In an sich bekannter Weise wurden Gelatinesteckkapseln folgender Zusammensetzung hergestellt:

| | |
|---|---|
| Paracetamol | 250,0 mg |
| Propyphenazon | 150,0 mg |
| Hydroxypropylmethylcellulose 2910/3 cP | 8,0 mg |
| Hydroxypropylmethylcellulose 2910/15 cP | 4,0 mg |
| POLYPLASDONE XL® | 10,0 mg |
| Maisstärke weiss | 42,0 mg |
| AVICEL PH-102® | 30,0 mg |
| Magnesiumstearat | 6,0 mg |
| Kapselgewicht | 500,0 mg |

**Patentansprüche**

1. Coffein freies analgetisches Präparat bestehend aus Paracetamol und Propyphenazon nebst üblichen Hilfsstoffen.

2. Präparat nach Anspruch 1, gekennzeichnet durch ein Gewichtsverhältnis von Paracetamol zu Propyphenazon von 5 zu 3.

3. Oral verabreichbares Präparat nach Anspruch 1 oder 2.

**Claims**

1. A caffeine-free analgesic preparation consisting of paracetamol and propyphenazone in addition to usual adjuvants.

2. A preparation according to claim 1, characterized by a weight ratio of paracetamol to propyphenazone of 5 to 3.

3. An orally administerable preparation according to claim 1 or 2.

**Revendications**

1. Préparation analgésique dépourvue de caféine, constituée de paracétamol et de propyphénazone, outre les additifs habituels.

2. Préparation selon la revendication 1, caractérisée par un rapport pondéral du paracétamol à la propyphénazone de 5 à 3.

3. Préparation administrable par voie orale selon la revendication 1 ou 2.

3